Europäisches Patentamt

European Patent Office

Office européen des brevets

(11) Veröffentlichungsnummer: **0 247 296 B1**

# EUROPÄISCHE PATENTSCHRIFT

(45) Veröffentlichungstag der Patentschrift: **28.08.91**

(51) Int. Cl.⁵: **A61N 1/36**, A61B 5/00

(21) Anmeldenummer: **87102949.2**

(22) Anmeldetag: **02.03.87**

(54) **Messvorrichtung zur intrakardialen Erfassung der Blutsauerstoffsättigung.**

(30) Priorität: **22.05.86 DE 3617180**

(43) Veröffentlichungstag der Anmeldung:
**02.12.87 Patentblatt 87/49**

(45) Bekanntmachung des Hinweises auf die
Patenterteilung:
**28.08.91 Patentblatt 91/35**

(84) Benannte Vertragsstaaten:
**DE FR GB IT NL SE**

(56) Entgegenhaltungen:
**EP-A- 0 059 868**
**DE-A- 2 717 659**

(73) Patentinhaber: **Siemens Aktiengesellschaft**
**Wittelsbacherplatz 2**
**W-8000 München 2(DE)**

(84) Benannte Vertragsstaaten:
**DE FR GB IT NL**

Patentinhaber: **Siemens Elema AB**
**Röntgenvägen 2**
**S-171 95 Solna 1(SE)**

(84) Benannte Vertragsstaaten:
**SE**

(72) Erfinder: **Liess, Hans-Dieter, Prof. Dr. Ing.**
**Pfaffenkamer Strasse 5**
**W-8193 Muensing(DE)**
Erfinder: **Heinze, Roland, Dipl.-Ing.**
**Simbacherstrasse 9**
**W-8000 München 80(DE)**

## Beschreibung

Die Erfindung betrifft ein Verfahren zur intrakardialen Erfassung der Blutsauerstoffsättigung zur Frequenzregelung eines Herzschrittmachers mit einer Meßsonde, die einen Meßstrompfad mit einem Lichtsender und einen Lichtempfänger enthält, wobei der Lichtempfänger das vom Lichtsender ausgesandte und vom Blut reflektierte Licht empfängt, wobei über die Meßsonde eine Nutzsignalmessung und eine von der Blutreflexion unabhängige Referenzmessung durchgeführt wird, wobei die Meßsonde über zwei Leitungen an eine Auswerteschaltung angeschlossen ist, die die Meßsonde mit einem Strom bzw. einer Spannung beaufschlagt und die dadurch bei Nutzsignalmessung und bei Referenzmessung entstehenden Signale getrennt auswertet.

Ferner betrifft die Erfindung eine Meßvorrichtung zur Durchführung des Verfahrens. Ein derartiges Verfahren ist aus der DE-PS 31 52 963 bekannt. Dabei enthält die Meßsonde eine Kombination aus einer Leuchtdiode und einem Fototransistor, die derart parallel geschaltet sind, daß der Durchlaßstrom durch die Leuchtdiode additiv überlagert wird von dem durch Lichteinwirkung verursachten Strom durch den Fototransistor. Bei Ansteuerung der Meßsonde mit einem konstanten Strom oder einer konstanten Spannung löst das vom Blut abhängig von seiner Sauerstoffsättigung reflektierte Licht im Fototransistor einen Stromfluß aus, der eine Stromänderung bzw. Spannungsänderung an der Meßsonde bewirkt. Die durch die Lichtreflexion erzeugte Spannungs- bzw. Stromänderung wird in einer Auswerteschaltung durch Vergleich des Meßsignals bei angesteuerter Leuchtdiode und Fototransistor mit einem Referenzsignal ermittelt.

Dieses Referenzsignal wird dadurch gebildet, daß man über eine der Leuchtdiode antiparallel geschaltete Diode einen Impuls gleicher Spannung, aber umgekehrten Vorzeichens wie für die Nutzsignalmessung schickt. Dabei sind vorzugsweise die Kennlinie der Diode im Referenzkreis und die Kennlinie der Leuchtdiode identisch.

Bei dieser Meßvorrichtung kommt man also sowohl für die Nutzsignalmessung als auch für die Referenzmessung mit nur zwei elektrischen Zuleitungen aus. Dies ist deshalb von besonderer Bedeutung, weil diese Zuleitungen in Kathedern mit möglichst geringem Durchmesser und hoher Flexibilität untergebracht werden müssen und außerdem jede zusätzliche Leitung die Ausfallwahrscheinlichkeit erhöht.

Nachteilig bei der genannten Anordnung ist jedoch, daß zur Referenzmessung eine Umpolung des Meßsignals erforderlich ist. Damit ist beim üblichen Aufbau der Spannungsversorgung von Herzschrittmachern, bei denen ein Pol der Versorgungsspannung fest mit dem Gehäuse verbunden ist, ein nicht unbeträchtlicher Schaltungsaufwand verbunden. Ferner wird für die Referenzmessung derselbe Strom verbraucht wie für die Nutzsignalmessung.

Im Handel erhältlich sind ferner Geräte, bei denen zur Referenzmessung eine Infrarot-Leuchtdiode eingeschaltet wird, wobei der Empfänger auch während der Referenzmessung in Betrieb bleibt. Die Wellenlänge der Infrarot-Leuchtdiode wird dabei so gewählt, daß dabei die Reflexion des Blutes von seiner Sauerstoffsättigung unabhängig ist. Damit erhält man eine Referenzmessung, bei der auch Ablagerungen an der Meßsonde berücksichtigt werden.

Aufgabe der Erfindung ist es, ein Verfahren und eine Meßvorrichtung zur Durchführung des Verfahrens der eingangs genannten Art mit nur zwei elektrischen Zuleitungen so auszugestalten, daß eine Referenzmessung ohne Umpolung der Meßspannung möglich ist.

Diese Aufgabe wird in einer ersten Lösungsvariante dadurch gelöst, daß an die Meßsonde ein Strom bzw. eine Spannung einheitlicher Polarität angelegt wird und daß in der Meßsonde die Nutzsignalmessung gegenüber der Referenzmessung zeitlich versetzt durchgeführt wird. Damit ist eine Nutzsignalmessung und eine davon unabhängige Referenzmessung ohne Umpolung der Meßspannung möglich. Ferner ist nur ein gemeinsamer Meßimpuls für Referenzmessung und Nutzsignalmessung erforderlich. Damit treten nur einmal Einschwingvorgänge auf und dieser gemeinsame Meßimpuls kann kürzer gemacht werden als die bisherigen Meßimpulse für Referenzmessung und Nutzsignalmessung zusammen. Damit ist eine Stromersparnis verbunden.

In einer zweiten Lösungsvariante wird die Aufgabe dadurch gelöst, daß in der Meßsonde eine Referenzmessung durchgeführt wird, solange diese mit einem unter einem Grenzwert liegenden Strom bzw. Spannung beaufschlagt ist und daß in der Meßsonde eine Nutzsignalmessung durchgeführt wird, sobald Strom bzw. Spannung diesen Grenzwert überschreiten. Auch hierbei ist eine Trennung von Nutzsignalmessung und Referenzmessung ohne Spannungs- bzw. Stromumkehr möglich, indem diese beiden Messungen mit unterschiedlichen Strömen durchgeführt werden. Dabei ist außerdem vorteilhaft, daß für die Referenzmessung nur eine geringere Stromstärke als für die Nutzsignalmessung benötigt wird.

In einer vorteilhaften Ausführungsform enthält der Meßstrompfad die Serienschaltung eines Widerstandes und einer lichtempfindlichen Diode, wobei dieser Serienschaltung ein Transistor parallel geschaltet ist, dessen Basis an den Verbindungspunkt von Widerstand und lichtempfindlicher Diode angeschlossen ist. Dabei reicht schon die Kapazität der lichtempfindlichen Diode aus, um das Einschalten des Transistors und damit des Meßstromkreises zu verzögern.

Bei einer vorteilhaften Ausführungsform verbindet ein über ein Verzögerungsglied angesteuerter Umschalter vor Ablauf einer Verzögerungszeit eine Infrarot-Leuchtdiode und nach Ablauf der Verzögerungszeit die Leuchtdiode mit den Eingangsklemmen der Meßsonde, wobei das Verzögerungsglied auf das Anlegen einer Spannung oder eines Stromes an die Meßsonde anspricht und der Meßstrompfad bei beiden Stellungen des Umschalters eingeschaltet bleibt. Damit kann auf einfache Weise die Referenzmessung mit Hilfe der Infrarot-Leuchtdiode durchgeführt werden.

Eine einfache Schaltungsanordnung ergibt sich, wenn in Reihe zu einem während der Referenzmessung aktiven Referenzstrompfad ein erster Schalter und in Reihe zum Meßstrompfad ein zweiter Schalter liegt, wobei der erste Schalter geschlossen und der zweite Schalter geöffnet wird, wenn die Meßsonde mit einem niedrigen Strom beaufschlagt wird, wobei der zweite Schalter geschlossen wird, wenn die Meßsonde mit einem höheren Strom beaufschlagt wird und wobei die Auswerteschaltung zur Referenzmessung die Meßsonde mit einem niedrigen Strom und zur Nutzsignalmessung mit einem höheren Strom gleicher Stromrichtung beaufschlagt.

Bei einer Anordnung mit Referenzmessung über eine Infrarot-Leuchtdiode kann die Meßsonde die Parallelschaltung einer Infrarot-Leuchtdiode, einer Leuchtdiode und eines Meßstrompfades enthalten, wobei in Reihe zur Infrarot-Leuchtdiode ein erster Schalter und in Reihe zur Leuchtdiode ein zweiter Schalter liegt, wobei der erste Schalter geschlossen und der zweite Schalter geöffnet wird, wenn die Meßsonde mit einem niedrigen Strom beaufschlagt wird und der zweite Schalter geschlossen wird, wenn die Meßsonde mit einem höheren Strom beaufschlagt wird und wobei die Auswerteschaltung zur Referenzmessung die Meßsonde mit einem niedrigen Strom und zur Nutzsignalmessung mit einem höheren Strom gleicher Stromrichtung beaufschlagt.

Eine bipolare EKG-Messung ist möglich, wenn die Meßsonde in der zweipoligen Zuleitung zu einer Elektrodenanordnung mit einer Stimulationselektrode und einer indifferenten Elektrode liegt, so daß diese Elektrodenanordnung der Meßsonde parallel liegt und wenn in der Verbindungsleitung zu einer der beiden Elektroden ein Schalter liegt, der geöffnet wird, sobald die Meßsonde von der Auswerteschaltung mit Spannung beaufschlagt wird. Dieser Schalter ist zweckmäßigerweise ein n-Kanal-Feldeffekttransistor, dessen Drain-Source-Strecke in der Zuleitung zur indifferenten Elektrode liegt und dessen Gate über einen Schwellwertschalter gesteuert wird, der die an der Meßsonde anstehende Spannung überwacht.

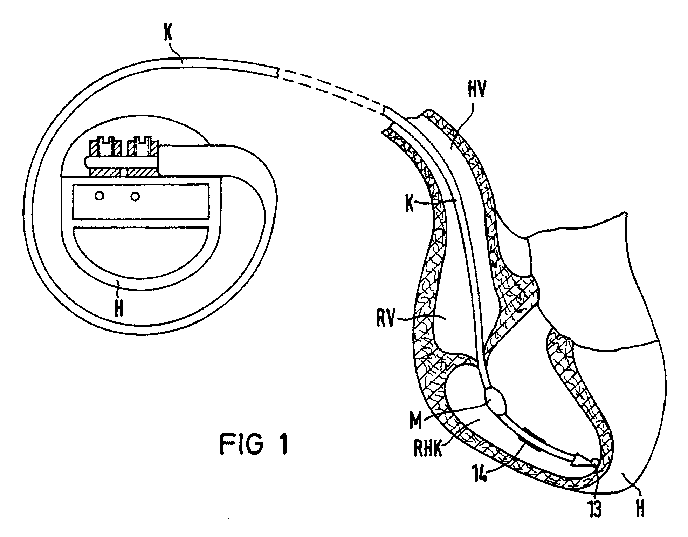

Ein Ausführungsbeispiel der Erfindung wird nachfolgend anhand der Figuren 1 bis 7 näher erläutert.

Fig. 1 zeigt die Anordnung eines Herzschrittmachers H, dessen zwei elektrische Leitungen enthaltender Katheder K in die obere Hohlvene HV durch den rechten Vorhof RV in die rechte Herzkammer RHK des Herzens H geführt wird, wo die Meßsonde M zur Messung der Blutsauerstoffsättigung plaziert wird und durch eine Stimulationselektrode 14 der Herzmuskel angeregt wird.

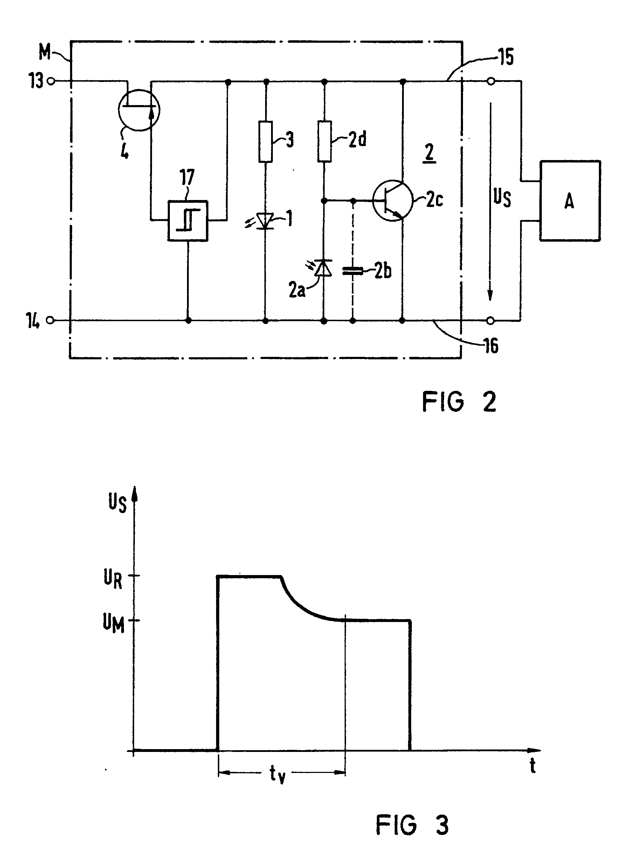

Ein Ausführungsbeispiel einer ersten erfindungsgemäßen Lösung für die Meßsonde M ist in Fig. 2 dargestellt. In der Meßsonde M sind die Reihenschaltung einer Leuchtdiode 1 als Lichtsender und eines Widerstands 3, die Reihenschaltung eines Widerstands 2d und einer lichtempfindlichen Diode 2a als Lichtempfänger sowie ein Transistor 2c parallel an die Zuleitungen anschlossen. Dabei sind die Leitrichtungen der Leuchtdiode 1 und der lichtempfindlichen Diode 2a entgegengesetzt gerichtet. Der Verbindungspunkt des Widerstands 2d und der lichtempfindlichen Diode 2a ist mit der Basis des Transistors 2c verbunden.

Wenn die Meßsonde von der im Herzschrittmacher angeordneten Auswerteschaltung A mit einem Spannungs- oder Stromimpuls beaufschlagt wird, so fließt zunächst nur über die als Referenzstrompfad dienende Reihenschaltung des Widerstands 3 und der Leuchtdiode 1 Strom, so daß die anstehende Meßspannung $U = U_R$ nur von der Leuchtdiode 1, dem Widerstand 3 und den Zuleitungswiderständen abhängig ist. Der Transistor 2c ist nämlich beim Einschalten noch gesperrt, da der gestrichelt gezeichnete Kondensator 2b noch nicht geladen ist. Dieser Kondensator 2b kann einfach durch die interne Kapazität der lichtempfindlichen Diode 2a gebildet sein. Erst wenn nach einer Verzögerungszeit $t_v$ der Kondensator 2b über den Widerstand 2d aufgeladen ist, wird der Transistor 2c leitend und bestimmt - falls die Meßsonde M mit einem eingeprägten Strom beaufschlagt wird - die Meßspannung $U_M$. Der entsprechende Spannungsverlauf ist in Fig. 3 dargestellt.

Die Leitfähigkeit des Transistors 2c ist von der Leitfähigkeit der lichtempfindlichen Diode 2a abhängig. Die lichtempfindliche Diode 2a ist so angeordnet, daß sie das von der Leuchtdiode 1 ausgesandte und vom Blut in Abhängigkeit von dessen Sauerstoffsättigung reflektierte Licht empfängt. Damit stellt also die Meßspannung $U_M$ ein Maß für die Sauerstoffsättigung des Blutes dar. Allerdings ist die Meßspannung $U_M$ auch von dem Widerstand der Verbindungsleitungen und der Temperatur der Meßsonde M abhängig. Dieser Fehler kann jedoch mit Hilfe der vorher ermittelten Referenzspannung $U_R$, in die diese Größen ebenfalls eingehen, kompensiert werden, indem die Differenz $\Delta U_F = U_R - U_M$ gebildet wird. Diese Differenz $\Delta U_F$ stellt dann das eigentliche Meßsignal dar, das nach der in der bereits eingangs genannten DE-PS 31 52 963 beschriebenen Weise ausgewertet werden kann.

In analoger Weise kann die Meßsonde auch mit einer eingeprägten Spannung beaufschlagt werden, wobei dann der Strom als Meßgröße dient.

In der Ausführungsform nach Fig. 2 ist in die Verbindungsleitung 15 zur indifferenten Elektrode 13 ein Feldeffekttransistor 4 eingefügt. Das Gate dieses Feldeffekttransistors 4 ist mit einer Schwellwertschaltung 17 verbunden, die die an der Meßsonde M anstehende Spannung überwacht. Sobald die Auswerteschaltung A die Meßsonde M mit einer Spannung beaufschlagt, sperrt der Feldeffekttransistor 4, so daß die indifferente Elektrode 13 von der Verbindungsleitung 15 abgetrennt ist. Damit wird folgender Vorteil erreicht:

Eine Zweifach-Elektrodenanordnung, nämlich eine indifferente Elektrode 13 und eine Stimulationselektrode 14 ist vorteilhaft, wenn man eine störungsfreie EKG-Erfassung im Herzen durchführen will. Wenn man jedoch die indifferente Elektrode 13 bei einem Meßvorgang der Meßsonde M nicht abtrennen würde, so hätte die Spannungsbeaufschlagung der Meßsonde M durch die Auswerteschaltung A stets auch einen unerwünschten Anregeimpuls für das Herz zur Folge. Dies wird bei der dargestellten Schaltung dadurch vermieden, daß bei Spannungsbeaufschlagung der Meßsonde M die indifferente Elektrode 13 von der Meßsonde abgetrennt ist.

Außerdem wird dadurch vermieden, daß der durch das Körpergewebe gebildete Widerstand zwischen der indifferenten Elektrode 13 und der Stimulationselektrode 14 das Meßsignal der Meßsonde M verfälscht.

Eine Störung der EKG-Messung durch die Meßsonde tritt nicht auf, da die EKG-Spannungen unter den Schwellspannungen der Schaltung der Meßsonde liegen.

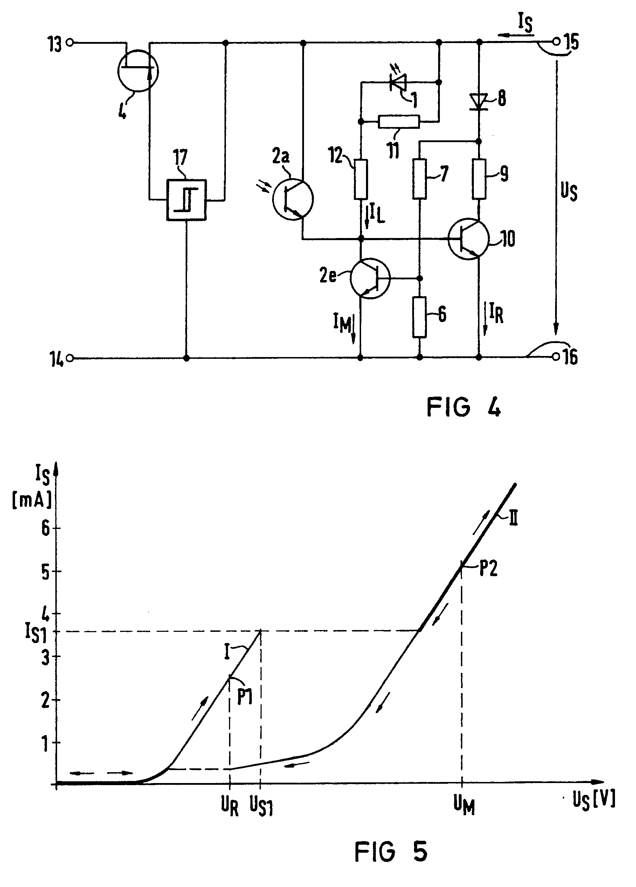

Ein Ausführungsbeispiel, das nach der zweiten Lösungsvariante arbeitet, ist in Figur 4 dargestellt. Fig. 5 zeigt die Abhängigkeit des Stromes $J_s$ in die Meßsonde M von der angelegten Spannung $U_s$ für diese Anordnung. Dabei ist zwischen den Anschlußleitungen 15 und 16 die Reihenschaltung einer Diode 8, eines Widerstands 9 und eines Transistors 10 angeschlossen. Ferner ist an diese Verbindungsleitung in Reihe die Parallelschaltung einer Leuchtdiode 1 mit einem Widerstand 11, ein Widerstand 12 und ein Transistor 2e angeschlossen. Ein Fototransistor 2a ist zwischen die Verbindungsleitung 15 und den Verbindungspunkt von Widerstand 12 und Transistor 2e eingefügt. Dieser Verbindungspunkt ist ferner mit der Basis des Transistors 10 verbunden. Zwischen den Verbindungspunkten der Diode 8 und des Widerstands 9 und der Verbindungsleitung 16 ist ein Spannungsteiler mit den Widerständen 6 und 7 angeschlossen, dessen Abgriff mit der Basis des Transistors 2e verbunden ist.

Wenn der über die Verbindungsleitungen 15 und 16 fließende Strom $I_S$ ansteigt, so wird zunächst über die Widerstände 11 und 12 der Transistor 10 leitend geschaltet, während der Transistor 2e noch sperrt. Daher bestimmt der Strompfad über die Diode 8, den Widerstand 9 und den Transistor 10 die an der Meßsonde anstehende Spannung. Der dadurch entstehende Teil der Strom-Spannungskennlinie ist in dem Diagramm nach Fig. 5 mit I bezeichnet. Der Strompfad mit der Diode 8, dem Widerstand 9 und dem Transistor 10 dient als Referenzstrompfad, wobei die Referenzmessung z.B. an einem in Fig. 5 mit P1 bezeichneten Arbeitspunkt erfolgt. Mit dieser Referenzmessung wird zunächst der Widerstand der Zuleitungen und die Temperatur der Meßsonde erfaßt.

Wenn die an der Meßsonde anstehende Spannung $U_S$ weiter steigt, wird über den Spannungsteiler mit den Widerständen 6, 7 der Transistor 2e leitend geschaltet. Der dabei vorliegende Spannungswert $U_{S1}$ bzw. Stromwert $I_{S1}$ ist durch das Teilerverhältnis der Widerstände 6 und 7 und durch den Widerstandswert des Widerstands 9 definiert. Sobald der Transistor 2e einschaltet, sperrt der Transistor 10, da seine Basis-Emitter-Spannung kurzgeschlossen wird. Damit wird der der Meßsonde zugeführte Strom $I_S$ vom Referenzstrompfad auf den Meßstrompfad mit der Leuchtdiode 1 und dem Fototransistor 2a umgeschaltet. Es gilt nunmehr der in Fig. 5 mit II bezeichnete Kennlinienteil. Die Schaltung zeigt dabei eine Hysterese, d.h. bei sinkendem Strom $I_s$ wird erst bei wesentlich kleineren Werten auf den Referenzkreis zurückgeschaltet als bei steigendem Strom, wie in Fig. 5 deutlich sichtbar ist.

Nach Umschaltung auf den Meßkreis kann nun wieder der Meßstrom bzw. die Meßspannung gewonnen werden, da die Leitfähigkeit des Fototransistors 2a von dem Blutsauerstoff abhängigen Anteil des reflektierten Lichts der Leuchtdiode 1 abhängig ist. Die Messung erfolgt dabei z.B. um einen in Fig. 5 mit P2 bezeichneten Arbeitspunkt. Wie bereits bei der erläuterten Anordnung wird die vorangehende Referenzmessung in der Auswerteschaltung zur Korrektur der Temperatur- und Zuleitungswiderstandseinflüsse verwendet.

Wie bei der in Fig. 2 dargestellten Anordnung kann auch hier in die Zuleitung 15 zur indifferenten Elektrode 13 ein Feldeffekttransistor 4 als Schalter zur Abtrennung der indifferenten Elektrode 13 während des Meßvorgangs angeordnet werden.

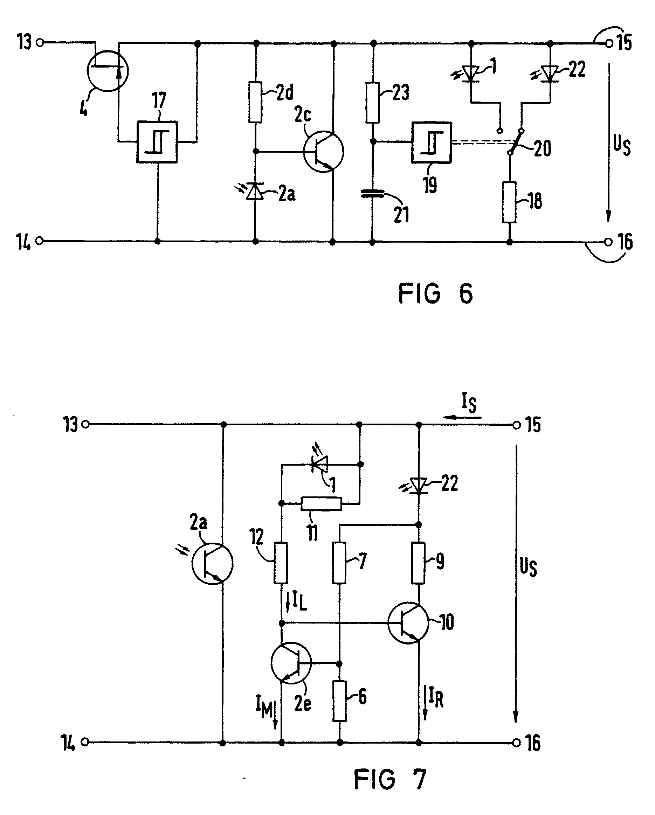

In Fig. 6 ist ein Ausführungsbeispiel dargestellt, bei dem eine Referenzmessung mit Hilfe einer Infrarot-Leuchtdiode 22 folgt. Dabei ist über einen Widerstand 18 und einen Umschalter 20 wahlweise eine Leuchtdiode 1 oder eine Infrarot-Leuchtdiode 22 an die Zuleitungen 15 und 16 anschließbar.

Ferner ist an die Zuleitungen 15 und 16 ein RC-Glied mit einem Kondensator 21 und einem Widerstand 23 in Reihenschaltung angeschlossen, wobei der Kondensator 21 mit der Zuleitung 16 verbunden ist. An den Abgriff des RC-Gliedes 20, 23 ist ein Schwellwertschalter 19 angeschlossen, der den Umschalter 20 steuert. Ferner ist an die Zuleitungen 15 und 16 ein Meßkreis angeschlossen, der aus einem Transistor 2c und einer diesem parallel geschalteten Reihenschaltung eines Widerstandes 2d und einer lichtempfindlichen Diode 2a besteht. Der Verbindungspunkt von Widerstand 2d und lichtempfindlicher Diode 2a ist mit der Basis des Transistors 2c verbunden. Schließlich enthält die Schaltung nach Figur 6 noch einen Schwellwertschalter 17 und einen Feldeffekttransistor 4, deren Funktion der Schaltung nach Figur 2 entspricht und daher hier nicht näher erläutert wird.

Wenn die Meßsonde mit einem Strom- oder Spannungsimpuls beaufschlagt wird, so steht der Umschalter 20 zunächst in der eingeschalteten Stellung. Damit leuchtet die Infrarot-Leuchtdiode 22, das ausgesandte Infrarot-Licht wird von der lichtempfindlichen Diode 2a empfangen und entsprechend der Leitfähigkeit der Diode 2a wird der Transistor 2c angesteuert.

Die Wellenlänge des Infrarot-Lichtes wird dabei so gewählt, daß die Reflexion von der Blutsauerstoffsättigung unabhängig ist. Damit erhält man ein Referenzsignal, in das der Zuleitungswiderstand, die Temperatur der Anordnung und durch eventuelle Ablangerungen an der Meßsonde hervorgerufene Reflexionen eingehen.

Mit der Beaufschlagung der Meßsonde M mit einem Strom-bzw. Spannungsmimpuls wird außerdem das aus dem RC-Glied 23, 21 und dem Schwellwertglied 19 bestehende Zeitglied angestoßen, das nach Ablauf einer vorgegebenen Verzögerungszeit den Umschalter 20 umschaltet. Damit ist nun die Leuchtdiode 1 in Betrieb, deren reflektiertes Licht wiederum von der lichtempfindlichen Diode 2a empfangen wird.

Wie bei den vorhergehend beschriebenen Anordnungen hat das von der Leuchtdiode 1 ausgesandte Licht eine Wellenlänge, bei der die Reflexion von der Blutsauerstoffsättigung abhängig ist, so daß hiermit eine Nutzsignalmessung durchgeführt wird. Durch Vergleich mit der Referenzmessung können die vorher genannten Parameter (Zuleitungswiderstand, Temperatur der Anordnung und Reflexion durch Ablagerungen) eliminiert werden.

In Figure 7 ist schließlich ein weiteres Ausführungsbeispiel dargestellt, bei dem ähnlich wie bei der Ausführungsform nach Figur 4 Referenzmessung und Nutzsignalmessung durch die Höhe der angelegten Spannung bzw. des eingespeisten Stromes unterschieden werden. Im Vergleich zur Anordnung nach Figur 4 ist lediglich die Diode 8 durch eine Infrarot-Diode 22 ersetzt. Ferner ist beim Ausführungsbeispiel nach Figure 7 der das reflektierte Licht empfangende Fototransistor 2a direkt zwischen den Anschlußleitungen 15 und 16 angeschlossen.

Die Umschaltung von der bei der Referenzmessung betriebenen Infrarot-Leuchtdiode 22 auf die bei der Nutzsignalmessung betriebene Leuchtdiode 1 erfolgt wie in Zusammenhang mit Figur 4 bereits beschrieben, so daß sich hier eine weitere Beschreibung erübrigt.

Dabei wird - im Unterschied zur Ausführungsform nach Figur 4 - auch während der Referenzmessung das von der Infrarot-Leuchtdiode 22 ausgesandte und vom Fototransistor 2a empfangene Licht ausgewertet, um wie bei Ausführungsbeispiel nach Figur 6 auch Reflexionen durch Ablagerungen auf der Meßsonde M bei der Referenzmessung zu berücksichtigen.

## Patentansprüche

1. Verfahren zur intrakardialen Erfassung der Blutsauerstoffsättigung zur Frequenzregelung eines Herzschrittmachers mit einer Meßsonde (M), die für eine Nutzsignalmessung einen Meßstrompfad mit einem Lichtsender (1) und einem Lichtempfänger (2a) enthält, wobei der Lichtempfänger (2a) das vom Lichtsender (1) ausgesandte und vom Blut reflektierte Licht empfängt, und wobei die Meßsonde (M) ferner einen Referenzstrompfad enthält, über den eine von der Blutreflexion unabhängige Referenzmessung durchgeführt wird, wobei die Meßsonde (M) mit Meßstrompfad und Referenzstrompfad über zwei Leitungen an eine Auswerteschaltung angeschlossen ist, die die Meßsonde mit einem Strom bzw. mit einer Spannung beaufschlagt und die dadurch bei Nutzsignalmessung und bei Referenzmessung entstehenden Signale getrennt auswertet, **dadurch gekennzeichnet,** daß in der Meßsonde (M) ein Strom bzw. eine Spannung einheitlicher Polarität angelegt wird und daß in der Meßsonde der zugeführte Strom zeitlich versetzt zur Nutzsignalmessung und zur Referenzmessung verwendet wird.

2. Verfahren zur intrakardialen Erfassung der Blutsauerstoffsättigung zur Frequenzregelung eines Herzschrittmachers mit einer Meßsonde (M), die für eine Nutzsignalmessung einen Meßstrompfad mit einem Lichtsender (1) und einem Lichtempfänger (2a) enthält, wobei der Lichtempfänger (2a) das vom Lichtsender (1) ausgesandte und vom Blut reflektierte Licht empfängt, und wobei die Meßsonde (M) ferner einen Referenzstrompfad enthält, über den eine von der Blutreflexion unabhängige Referenzmessung durchgeführt wird, wobei die Meßsonde (M) mit Nutzstrompfad und Referenzstrompfad über zwei Leitungen an eine Auswerteschaltung (A) angeschlossen ist, die die Meßsonde mit einem Strom bzw. mit einer Spannung beaufschlagt und die dadurch bei Nutzsignalmessung und bei Referenzmessung entstehenden Signale getrennt auswertet, **dadurch gekennzeichnet,** daß in der Meßsonde (M) eine Referenzmessung durchgeführt wird, solange diese mit einem unter einem Grenzwert ($I_{S1}$) liegenden Strom bzw. Spannung beaufschlagt ist und daß in der Meßsonde (M) eine Nutzsignalmessung durchgeführt wird, sobald Strom bzw. Spannung diesen Grenzwert überschreiten.

3. Meßvorrichtung zur Durchführung des Verfahrens nach Anspruch 1, **dadurch gekennzeichnet,** daß der Meßstrompfad (2) die Serienschaltung eines Widerstands (2d) und einer lichtempfindlichen Diode (2a) enthält und daß dieser Serienschaltung ein Transistor (2c) parallel geschaltet ist, dessen Basis an den Verbindungspunkt von Widerstand (2d) und lichtempfindlicher Diode (2a) angeschlossen ist.

4. Meßvorrichtung nach Anspruch 3, **dadurch gekennzeichnet,** daß ein zur Referenzmessung benutzter Referenzstrompfad die Reihenschaltung eines Widerstands (3) und einer Leuchtdiode (1) enthält.

5. Meßvorrichtung nach Anspruch 3, **dadurch gekennzeichnet,** daß ein über ein Verzögerungsglied (19 bis 21) angesteuerter Umschalter (20) vor Ablauf einer Verzögerungszeit eine Infrarot-Leuchtdiode (22) und nach Ablauf der Verzögerungszeit die Leuchtdiode (1) mit den Eingangsklemmen der Meßsonde verbindet, daß das Verzögerungsglied (19 bis 21) auf das Anlegen einer Spannung oder eines Stromes an die Meßsonde (M) anspricht und daß der Meßstrompfad bei beiden Stellungen des Umschalters (20) eingeschaltet bleibt.

6. Meßvorrichtung zur Durchführung des Verfahrens nach Anspruch 2, **dadurch gekennzeichnet,** daß in Reihe zu einem während der Referenzmessung aktiven Referenzstrompfad (8, 9) ein erster Schalter (10) und in Reihe zum Meßstrompfad (2a) ein zweiter Schalter (2e) liegt, daß der erste Schalter (10) geschlossen und der zweite Schalter (2e) geöffnet wird, wenn die Meßsonde mit einem niedrigen Strom ($I_s$) beaufschlagt wird, daß der zweite Schalter (2e) geschlossen wird, wenn die Meßsonde (M) mit einen höheren Strom ($I_s$) beaufschlagt wird und daß die Auswerteschaltung zur Referenzmessung die Meßsonde mit einem niedrigen Strom ($I_s$) und zur Nutzsignalmessung mit einen höheren Strom ($I_s$) gleicher Stromrichtung beaufschlagt.

7. Meßvorrichtung zur Durchführung des Verfahrens nach Anspruch 2, **dadurch gekennzeichnet,** daß die Meßsonde die Parallelschaltung einer Infrarot-Leuchtdiode (22), einer Leuchtdiode (1) und eines Meßstrompfades (2a) enthält, daß in Reihe zur Infrarot-Leuchtdiode (22) ein erster Schalter (10) und in Reihe zur Leuchtdiode (1) ein zweiter Schalter (2e) liegt, daß der erste Schalter (10) geschlossen und der zweite Schalter (2e) geöffnet wird, wenn die Meßsonde mit einem niedrigen Strom ($I_s$) beaufschlagt wird, daß der zweite Schalter (2e) geschlossen wird, wenn die Meßsonde (M) mit einem höheren Strom ($I_s$) beaufschlagt wird und daß die Auswerteschaltung (A) zur Referenzmessung die Meßsonde (M) mit einem niedrigen Strom ($I_s$) und zur Nutzsignalmessung mit einem höheren Strom ($I_s$) gleicher Stromrichtung beaufschlagt.

8. Meßvorrichtung nach Anspruch 6 oder 7, **dadurch gekennzeichnet,** daß der zweite Schalter (2e) ein Transistor ist, dessen Basis mit dem Abgriff eines Spannungsteilers (6,7) verbunden ist, der der Reihenschaltung eines Widerstandes (9) und des ersten Schalters (10) parallel geschaltet ist.

9. Meßvorrichtung nach einem der Ansprüche 6 bis 8, **dadurch gekennzeichnet,** daß der erste Schalter (10) ein Transistor ist, dessen Basis mit dem Verbindungspunkt der Leuchtdiode (1) mit dem zweiten Schalter (2e) verbunden ist.

10. Meßvorrichtung nach einem der Ansprüche 6 bis 9, **dadurch gekennzeichnet,** daß der Leuchtdiode (1) ein Widerstand (11) parallel und dieser Parallelschaltung ein Widerstand (12) in Serie geschaltet ist.

11. Meßvorrichtung nach einem der Ansprüche 3 bis 10, **dadurch gekennzeichnet,** daß die Meßsonde (M) in der zweipoligen Zuleitung zu einer Elektrodenanordnung mit einer Stimulationselektrode (14) und einer indifferenten Elektrode (13) liegt, so daß diese Elektrodenanordnung (13, 14) der Meßsonde (M) parallel liegt und daß in der Verbindungsleitung zu einer der beiden Elektroden (13, 14) ein Schalter (4) liegt, der geöffnet wird, sobald die Meßsonde von der Auswerteschaltung (A) mit Spannung beaufschlagt wird.

12. Meßvorrichtung nach Anspruch 11, **dadurch gekennzeichnet,** daß der Schalter (4) ein n-Kanal-Feldeffekttransistor ist, dessen Drain-Source-Strecke in der Zuleitung zur indifferenten Elektrode (13) liegt und dessen Gate über einen Schwellwertschalter (17) gesteuert wird, der die an der Meßsonde (M) anstehende Spannung überwacht.

## Claims

1. Method for the intracardiac detection of blood oxygen saturation for the frequency regulation of a heart pace maker with a measuring probe (M) which, for a useful signal measurement, comprises a measuring current path with a light emitter (1) and a light receiver (2a), with the light receiver (2a) receiving the light emitted from the light emitter (1) and reflected by the blood, and with the measuring probe (M) further comprising a reference current path by way of which a reference measurement, independent of the blood reflection, is carried out, the measuring probe (M) with the measuring current path and the reference current path being connected by way of two leads to an evaluation circuit which applies a current or a voltage to the measuring probe and evaluates separately the signals thus occurring during the useful signal measurement and the reference measurement, **characterised in that** a current or a voltage of uniform polarity is applied in the measuring probe (M), and in that, in the measuring probe, the current supplied is used in chronologically offset manner for the useful signal measurement and for the reference measurement.

2. Method for the intracardiac detection of blood oxygen saturation for the frequency regulation of a heart pace maker with a measuring probe (M) which, for a useful signal measurement, comprises a measuring current path with a light emitter (1) and a light receiver (2a), with the light receiver (2a) receiving the light emitted from the light emitter (1) and reflected by the blood, and with the measuring probe (M) further comprising a reference current path by way of which a reference measurement, independent of the blood reflection, is carried out, the measuring probe (M) with the measuring current path and the reference current path being connected by way of two leads to an evaluation circuit (A) which applies a current or a voltage to the measuring probe and evaluates separately the signals thus occurring during the useful signal measurement and the reference measurement, **characterised in that,** in the measuring probe (M), a reference measurement is carried out as long as a current or voltage below a limit value ($I_{S1}$) is applied to the measuring probe, and in that, in the measuring probe (M), a useful signal measurement is carried out as soon as current or voltage exceeds this limit value.

3. Measuring device for carrying out the method according to claim 1, **characterised in that** the measuring current path (2) comprises a series circuit consisting of a resistor (2d) and a light-sensitive diode (2a), and in that a transistor (2c) is connected in parallel with this series circuit, the base of this transistor being connected to the connection point of the resistor (2d) and the light-sensitive diode (2a).

4. Measuring device according to claim 3, **characterised in that** a reference current path used for the reference measurement comprises a series circuit consisting of a resistor (3) and a light-emitting diode (1).

5. Measuring device according to claim 3, **characterised in that** a changeover switch (20) activated by way of a delay element (19 to 21) connects an infrared light-emitting diode (22) to the input terminals of the measuring probe before the expiry of a delay time, and connects the light-emitting diode (1) to the input terminals of the measuring probe after the expiry of the delay time, and in that the delay element (19 to 21) responds to the application of a voltage or a current to the measuring probe (M), and in that the measuring current path remains switched on in both positions of the changeover switch (20).

6. Measuring device for carrying out the method according to claim 2, **characterised in that** a first switch (10) is connected in series with a reference current path (8, 9) which is active during the reference measurement, and a second switch (2e) is connected in series with the measuring current path (2a), and in that the first switch (10) is closed and the second switch (2e) opened when a low current ($I_S$) is applied to the measuring probe, and in that the second switch (2e) is closed when a higher current ($I_s$) is applied to the measuring probe (M), and in that the evaluation circuit applies a low current ($I_s$) to the measuring probe for the reference measurement and applies a higher current ($I_s$) of the same current direction to the measuring probe for the useful signal measurement.

7. Measuring device for carrying out the method according to claim 2, **characterised in that** the measuring probe comprises a parallel circuit consisting of an infrared light-emitting diode (22), a light-emitting diode (1) and a measuring current path (2a), and in that a first switch (10) is connected in series with the infrared light-emitting diode (22), and a second switch (2e) is connected in series with the light-emitting diode (1), and in that the first switch (10) is closed and the second switch (2e) is opened when a low current ($I_s$) is applied to the measuring probe, and in that the second switch (2e) is closed when a higher current ($I_s$) is applied to the measuring probe (M), and in that the evaluation circuit (A) applies a low current ($I_s$) to the measuring probe (M) for the reference measurement and applies a higher current ($I_s$) with the same direction of current to the measuring probe for the useful signal measurement.

8. Measuring device according to claim 6 or 7, **characterised in that** the second switch (2e) is a transistor, the base of which is connected to the tap of a voltage divider (6, 7) which is connected in parallel with a series circuit consisting of a resistor (9) and the first switch (10).

9. Measuring device according to one of claims 6 to 8, **characterised in that** the first switch (10) is a transistor, the base of which is connected to the connection point of the light-emitting diode (1) and the second switch (2e).

10. Measuring device according to one of claims 6 to 9, **characterised in that** a resistor (10) is connected in parallel with the light-emitting diode (1) and a resistor (12) is connected in series with this parallel circuit.

11. Measuring device according to one of claims 3 to 10, **characterised in that** the measuring probe (M) is connected in the bipolar supply lead to an electrode arrangement having a stimulation electrode (14) and a neutral electrode (13), so that this electrode arrangement (13, 14) is in parallel with the measuring probe (M), and in that, in the connection lead to one of the two electrodes (13, 14), there is a switch (4) which is opened as soon as the evaluation circuit (A) applies a voltage to the measuring probe.

12. Measuring device according to claim 11, **characterised in that** the switch (4) is an n-channel field effect transistor, the drain-source route of this switch (4) being located in the supply lead to the neutral electrode (13), and the gate of this switch (4) being controlled by way of a threshold switch (17) which monitors the voltage occurring at the measuring probe (M).

**Revendications**

1. Procédé de détermination intracardiaque de la saturation en oxygène dans le sang, pour régler la fréquence d'un stimulateur cardiaque comportant une sonde de mesure (M), qui comporte, pour une mesure du signal utile, une voie de mesure du courant comportant une source de lumière (1) et un récepteur de lumière (2a), et selon lequel le récepteur de lumière (2a) reçoit la lumière émise par la source de lumière (1) et réfléchie par le sang et la sonde de mesure (M) contient en outre une voie de circulation d'un courant de référence et au moyen de laquelle est exécutée une mesure de référence indépendante de la réflexion sur le sang, et selon lequel la sonde de mesure (M) équipée de la voie de circulation du courant de mesure et de la voie de circulation du courant de référence est raccordée par l'intermédiaire de deux conducteurs à un circuit d'évaluation, qui charge la sonde de mesure avec un courant ou avec une tension et évalue séparément les signaux obtenus de ce fait lors de la mesure du signal utile, lors de la mesure de référence, caractérisé par le fait qu'un courant ou une tension possédant une polarité uniforme est appliqué à la sonde de mesure (M) et que, dans la sonde de mesure, le courant envoyé est utilisé, en étant décalé dans le temps, pour la mesure du signal utile et pour la mesure de référence.

2. Procédé de détermination intracardiaque de la saturation en oxygène dans le sang, pour régler la fréquence d'un stimulateur cardiaque comportant une sonde de mesure (M), qui contient, pour une mesure du signal utile, une voie de mesure du courant comportant une source de lumière (1) et un récepteur de lumière (2a), et selon lequel le récepteur de lumière (2a) reçoit la lumière émise par la source de lumière (1) et réfléchie par le sang et la sonde de mesure (M) contient en outre une voie de circulation d'un courant de référence et au moyen de laquelle est exécutée une mesure de référence indépendante de la réflexion sur le sang, et selon lequel la sonde de mesure (M) équipée de la voie de circulation du courant de mesure et de la voie de circulation du courant de référence est raccordée par l'intermédiaire de deux conducteurs à un circuit d'évaluation qui charge la sonde de mesure avec un courant ou avec une tension et évalue séparément les signaux obtenus de ce fait lors de la mesure du signal utile, et lors de la mesure de référence, caractérisé par le fait que, dans la sonde de mesure (M), on exécute une mesure de référence tant que cette sonde est chargée par un courant ou une tension inférieure à une valeur limite ($I_{S1}$), et que, dans la sonde de mesure (M), on exécute une mesure du signal utile dès que le courant ou la tension dépasse cette valeur limite.

3. Dispositif de mesure pour la mise en oeuvre du procédé suivant la revendication 1, caractérisé par le fait que la voie (2) de circulation du courant de mesure contient le circuit série formé d'une résistance (2d) et d'une diode photosensible (2a), et qu'en parallèle avec ce circuit série est branché un transistor (2c), dont la base est raccordée au point de raccordement de la résistance (2d) et de la diode photosensible (2a).

4. Dispositif de mesure suivant la revendication 3, caractérisé par le fait qu'une voie de circulation du courant de référence, utilisé pour la mesure de référence, contient le circuit série formé d'une résistance (3) et d'une diode à luminescence (5).

5. Dispositif de mesure suivant la revendication 3, caractérisé par le fait qu'un commutateur (20) commandé par un circuit de retardement (19 à 21) raccorde, avant l'écoulement d'un retard, une diode à luminescence émettant dans l'infrarouge (22) et, après l'écoulement du retard, la diode à luminescence (1) aux bornes d'entrée de la sonde de mesure, que le circuit de retardement (19 à 21) répond à l'application d'une tension d'un courant à la sonde de mesure (M) et que la voie de circulation du courant de mesure reste fermée, lorsque le commutateur (20) est dans ses deux positions.

6. Dispositif de mesure pour la mise en oeuvre du procédé suivant la revendication 2, caractérisé par le fait qu'un premier interrupteur (10) est branché en série avec une voie de circulation du courant de référence (8,9), active pendant la mesure de référence, et qu'un second interrupteur (2e) est branché en série avec la voie de circulation du courant de mesure (2a), que le premier interrupteur (10) est fermé et le second interrupteur (2e) ouvert lorsque la sonde de mesure est chargée par un faible courant ($I_S$), que le second interrupteur (2e) est fermé lorsque la sonde de mesure (M) est chargée par un courant supérieur ($I_s$) et que le circuit d'évaluation charge la sonde de mesure avec un courant faible ($I_s$), pour la mesure de référence, et avec un courant supérieur ($I_s$) de même sens, pour la mesure du signal utile.

7. Dispositif de mesure pour la mise en oeuvre du procédé suivant la revendication 2, caractérisé par le fait que la sonde de mesure contient le circuit en parallèle formé d'une diode à luminescence émettant l'infrarouge (22), d'une diode à luminescence (1) et d'une voie de circulation du courant de mesure (2a), et qu'un premier interrupteur (10) est branché en série avec la diode à luminescence à infrarouge (22) et qu'un second interrupteur (2e) est branché avec la diode à luminescence (1), que le premier interrupteur (10) est fermé et que le second interrupteur (2e) ouvert lorsque la sonde de mesure est chargée avec un courant ($I_s$) faible, que le second interrupteur (2e) est fermé lorsque la sonde de mesure (M) est chargée par un courant ($I_s$) supérieur et que le circuit d'évaluation (A) charge la sonde de mesure (M) avec un courant ($I_s$) faible, pour la mesure de référence, et avec un courant ($I_s$) supérieur et de même sens pour la mesure du signal utile.

8. Dispositif du signal de mesure suivant la revendication 6 ou 7, caractérisé par le fait que le second interrupteur (2e) est un transistor, dont la base est raccordée à la prise d'un diviseur de tension (6,7), qui est branché en parallèle avec le circuit série formé d'une résistance (9) et du premier interrupteur (10).

9. Dispositif de mesure suivant l'une des revendications 6 à 8, caractérisé par le fait que le premier interrupteur (17) est un transistor, dont la base est raccordée au point de raccordement de la diode à luminescence (1) et du second interrupteur (2e).

10. Dispositif de mesure suivant l'une des revendications 6 à 9, caractérisé par le fait qu'une résistance (11) est branchée en parallèle avec la diode à luminescence (1) et qu'une résistance (12) est branchée en série avec ce circuit en parallèle.

11. Dispositif de mesure suivant l'une des revendications 3 à 10, caractérisé par le fait que la sonde de mesure (M) est située dans le conducteur d'alimentation bipolaire aboutissant à un dispositif à électrodes comprenant une électrode de stimulation (14) et une électrode indifférente (13), de sorte que ce dispositif à électrodes (13,14) est branché en parallèle avec la sonde de mesure (M) et que dans le conducteur de raccordement aboutissant à l'une des deux électrodes (13,14) est branché un interrupteur (4) qui est ouvert dès que la sonde de mesure est chargée avec une tension par le circuit d'évaluation (A).

12. Dispositif de mesure suivant la revendication 11, caractérisé par le fait que l'interrupteur (4) est un transistor à effet de champ à canal n, dont la section drain-source est située dans le conducteur d'alimentation aboutissant à l'électrode indifférente (13) et dont la grille est commandée par l'intermédiaire d'un commutateur à valeur de seuil (17), qui contrôle la tension appliquée à la sonde de mesure (M).

FIG 1

FIG 2

FIG 3

12

FIG 4

FIG 5

FIG 6

FIG 7